(19)

Europäisches
Patentamt
European
Patent Office
Office européen
des brevets

(11)     **EP 4 473 901 A1**

(12)     **EUROPEAN PATENT APPLICATION**

(43) Date of publication:
**11.12.2024   Bulletin 2024/50**

(21) Application number: **23185979.4**

(22) Date of filing: **18.07.2023**

(51) International Patent Classification (IPC):
**A61B 5/0205** (2006.01)     **A61B 5/024** (2006.01)
**A61B 5/11** (2006.01)       **A61B 5/00** (2006.01)
**G01R 33/567** (2006.01)     **A61B 5/055** (2006.01)

(52) Cooperative Patent Classification (CPC):
**A61B 5/1102; A61B 5/0205; A61B 5/02416;
A61B 5/1128; A61B 5/7292; G01R 33/5673;
A61B 5/055**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:   **07.06.2023   PCT/CN2023/098754**

(71) Applicant: **Koninklijke Philips N.V.
5656 AG Eindhoven (NL)**

(72) Inventors:
  • **DER SARKISSIAN, Henri Henroutune
    Eindhoven (NL)**

  • **DEN BRINKER, Albertus Cornelis
    Eindhoven (NL)**
  • **WUELBERN, Jan Hendrik
    5656AG Eindhoven (NL)**
  • **WEISS, Steffen
    Eindhoven (NL)**
  • **WENJIN, Wang
    Eindhoven (NL)**
  • **PAPPOUS, Ioannis
    Eindhoven (NL)**

(74) Representative: **Philips Intellectual Property &
Standards
High Tech Campus 52
5656 AG Eindhoven (NL)**

(54)     **TRIGGERING MEDICAL DATA ACQUISITION USING PPG AND BCG SIGNALS**

(57)     Disclosed herein is a method comprising: receiving (201) video frames of a video which have been acquired from a subject. A photoplethysmogram, PPG, signal and a ballistocardiogram, BCG, signal may be determined (203) from the video frames. First markers may be determined (205) in the PPG signal. Second markers may be determined (207) in the BCG signal. A selected first marker of the first markers and/or a selected second marker of the second markers may be used to define (209) a trigger time for acquiring medical data, such as MRI data, from the subject.

Fig. 2

**EP 4 473 901 A1**

**Description**

FIELD OF THE INVENTION

The invention relates to camera-based photoplethysmography systems, in particular to a determination of a trigger time for triggering the acquisition of medical data.

BACKGROUND OF THE INVENTION

In medical data acquisition systems, part(s) of the body are scanned. In order to reduce motion artifacts in these medical images, gated data acquisition is done where triggers for gating are generated based on the state of the target organ, e.g., for cardiac scanning an electrocardiogram (ECG) signal is acquired and the R-peak of the ECG signal can be used to determine the gating. However, there is a need for an alternative solution as an ECG measurement during a scanning operation may come with various disadvantages.

SUMMARY OF THE INVENTION

The invention provides for a system, a computer program, and a method in the independent claims. Embodiments are given in the dependent claims.

Embodiments provide a remote camera-based triggering system for a medical data acquisition system (e.g., magnetic resonance imaging (MRI) system or computerized tomography (CT) system). The system has means for separating ballistocardiogram (BCG) signal from photoplethysmogram (PPG) signal. By separating PPG signal from BCG signal, cleaner signals are obtained which may enable marker extraction in either signal with more fidelity. By separating both signals, the disadvantages when operating on either PPG or BCG signal may be mitigated. This may lead to better triggering of medical data acquisition. The relation (e.g., time offset) between markers in PPG and BCG signals can be determined. This can be done reliable at relatively low SNR by averaging or pooling of data over multiple cardiac cycles. The time offset may enable a seamless transition between trigger creation based on PPG signal or BCG signal.

In one aspect the invention provides for a system. The system may, for example, be a medical system. The system comprises: a memory storing machine executable instructions; and a computational system, wherein execution of the machine executable instructions causes the computational system to: receive video frames of a video which have been acquired from a subject; determine a PPG, signal and a BCG signal from the video frames; determine first markers in the PPG signal; determine second markers in the BCG signal; use a selected first marker of the first markers and/or a selected second marker of the second markers to define a trigger time for acquiring medical data from the subject. The medical data may, for example, be acquired from an organ e.g., heart, of the subject. The medical data may, for example, be MR data or CT data.

The video frames may be acquired by an optical imaging system. The optical imaging system may be any imaging device capable of acquiring a sequence of images, and preferably is one or more digital video cameras that output digital video frames with a time stamp annotating each frame. The image acquired by the optical imaging system may be a video frame. The optical imaging system may be remote from the subject. This may enable to measure vital sign information remotely e.g., without having to attach a sensor to the subject. For example, the optical imaging system may be configured to acquire the video frames from an imaging zone of the subject. The imaging zone may, for example, be the face of the subject. The video frames comprise a sequence of frames acquired at successive equally spaced points in time. The video frames may be acquired at a given frame rate. The frame rate refers to the number of frames per second. The optical imaging system may be configured to store and transmit the video frames.

The PPG signal and the BCG signal may be extracted from the received video frames. In addition, first markers may be determined in the PPG signal. The first markers may be referred to as PPG markers. Second markers may be determined in the BCG signal. The second markers may be referred to as BCG markers. The determined BCG markers may be specific data points in a time window of the BCG signal. The time window may, for example, represent one cardiac cycle of the subject. The BCG marker may, for example, be any one of: a peak point, an onset point or an offset point. The determined PPG markers may be specific data points in a time window of the PPG signal. The time window may, for example, represent one cardiac cycle of the subject. The PPG marker may, for example, be any one of: a peak point, an onset point or an offset point. The amount of first markers and second markers may, thus, be determined according to the time window length. The BCG marker may, for example, be created or determined by filtering and transforming the BCG signal. The filtering and transforming may, for example, comprise taking the derivative of the BCG signal and detecting the position of a peak point, an onset point, an offset point or a zero-crossing point and providing one of the points as the BCG marker. The PPG marker may, for example, be created or determined by filtering and transforming the PPG signal. The filtering and transforming may, for example, comprise taking the derivative of the PPG signal and detecting the position of a peak point, an onset point, an offset point or a zero-crossing point and providing one of the points as the PPG marker. Thus, the BCG markers which are determined in the BCG signal may, for example, comprise a same type or different types of points e.g., the BCG markers may comprise one or more peak points, one or more onset points etc. The PPG markers which are determined in the PPG signal may, for example, comprise a same type or different types of points e.g., the PPG markers may comprise one or more peak points, one or more onset points etc.

One or more BCG markers may be selected from the BCG markers. One or more PPG markers may be selected from the PPG markers. In the following, and for simplification of the description, the number of selected PPG markers may be one selected PPG marker and the number of selected BCG markers may be one selected BCG marker; however, it is not limited to. The selected BCG marker may represent a specific point of the cardiac cycle of the subject e.g., the selected BCG marker may represent the beginning of the diastole period or systole period.

The selection of the BCG marker(s) may be performed using a first selection criterion. The first selection criterion may require a maximum number of selected BCG markers e.g., the maximum number may be one. Alternatively, or additionally, the first selection criterion may require that the selected BCG marker is a predefined fiducial point e.g., the selected BCG marker may be a peak point. The selection of the PPG marker(s) may be performed using a second selection criterion. The second selection criterion may require a maximum number of selected PPG markers e.g., the maximum number may be one. Alternatively, or additionally, the second selection criterion may require that the selected PPG marker is a predefined fiducial point e.g., the selected PPG marker may be a zero-crossing point.

The selected one or more BCG markers may be used to determine the trigger time for acquiring the medical data from the subject. For example, the trigger time may be equal to the time associated with the selected BCG marker. Alternatively, selected one or more PPG markers may be used to determine the trigger time for acquiring the medical data from the subject. Alternatively, the selected one or more BCG markers and the selected one or more PPG markers may be used to determine the trigger time for acquiring the medical data from the subject.

In one another example, the trigger time may be the selected BCG marker plus a given time range "tau", where the time range tau may be calibrated for a specific cardiac phase of the subject or the time range tau may be a user defined time range.

In another example, a table may be provided per scan type, wherein the table comprises for a given BCG marker and a given Inter-Beat-Interval (IBI), the distance (in time) between the BCG marker and the pre-pulse trigger and/or data acquisition window. Thus, after selecting the BCG marker, the table may be used to extract the distance associated with the selected BCG marker and the IBI of the subject, and the extracted distance may be used as the trigger time. For example, the extracted distance may be a waiting period for acquiring data after the point of time of the selected BCG marker, wherein the waiting period may be the time the medical system has to wait to shoot the pre-pulse and the acquisition.

The present subject matter may enable a reliable acquisition of medical data based on accurately determined trigger times using remote camera-based measurements. The present subject matter may thus provide an improved alternative of the ECG-based triggering. Indeed, the camera-based measurements may comprise both PPG and BCG information. The BCG information can be used for triggering the acquisition of the medical data. Alternatively, the PPG information can be used for triggering acquisitions by extracting well-defined markers (e.g., per cardiac cycle). However, the PPG information may lack information on the state of the target organ (the heart). For that, the BCG information may be used in combination with PPG for triggering; because BCG contains information on the state of the target organ as it reflects the contraction of the muscles of the heart. Alternatively, statistical models may be used in combination with the PPG information to define the trigger time.

In one example, the trigger time may be determined using the selected PPG marker. In addition, the PPG marker may be associated with a specific point of the cardiac cycle of the subject using a temporal offset. This may further improve the triggering based on PPG markers compared to using the PPG marker only. The temporal offset may be used in combination with the selected PPG marker in order to determine the trigger time. For example, the selected PPG marker may be shifted by the temporal offset to obtain the trigger time. The temporal offset may, for example, be a pulse transit time (PTT) but it is not limited to, because the temporal offset between PPG and BCG signal may be more general as any marker could be target. The PTT may be defined as an offset between a marker (indicative for the arrival of the blood bolus) in the PPG signals obtained from different sites of the body.

The present subject matter may provide alternative offset determination techniques to provide the temporal offset. These offset determination techniques may enable to measure the temporal offset or predict the temporal offset. In case the temporal offset cannot be determined with one offset determination technique, the other offset determination technique may instated be used to determine the temporal offset. For example, in case the measurement of the temporal offset using BCG and PPG signals fails, the temporal offset may be predicted e.g., using a statistical model.

According to one example, the temporal offset may be determined one time and then reused for determining the trigger time multiple times. This may save resources that would otherwise be required by a repeated determination of the offset while still providing reliable trigger times. Alternatively, the temporal offset may be regularly updated or determined. For example, the temporal offset may be determined each time the PPG marker is selected from a given set of video frames. This may provide an accurate triggering of acquisition of medical data.

According to one example offset determination technique, BCG markers and PPG markers are compared to identify unique pairs of BCG marker and PPG marker which, each pair covering a time interval, wherein are the time interval is smaller than one cardiac cycle of the subject. The temporal offset may be determined using the time intervals of the identified pairs. A pair of BCG marker and PPG marker may be unique if it does not share any marker with any other identified pair of BCG marker and PPG marker. For example, the time intervals may be combined to obtain the temporal offset. The temporal offset may, for example, be the average of the time intervals. The temporal offset may be used in combination with the selected PPG marker in order to determine the trigger time. For example, the time of the selected PPG marker may be shifted by the temporal offset. The resulting shifted PPG marker may further be shifted by the time range tau or by the extracted distance associated with the identified BCG marker, the identified BCG marker which has a distance to the PPG marker equal to the temporal offset or equal to one of the time intervals.

According to one example offset determination technique, the PPG signal and the BCG signal may be segmented into cardiac cycles of the subject. Representative cycle waveforms may be generated from the segmented PPG signal and the segmented BCG signal respectively. The generated representative cycle waveforms may be used to determine or identify at least one pair of BCG and PPG markers, wherein the temporal offset may be determined using the determined pair of BCG and PPG markers. The temporal offset may be the time interval covered by (or between) the pair of BCG and PPG markers. For example, the representative cycle waveform computation for both PPG and BCG may deploy the same segmentation for cycle construction.

In one example, a graph extending over one cardiac cycle containing a PPG waveform may be obtained from the extracted PPG signal, wherein the identified PPG marker may be any peak point of the PPG waveform. A graph extending over one cardiac cycle containing a BCG waveform may be obtained from the extracted PPG signal, wherein the identified BCG marker may be any zero-crossing point of the BCG waveform. This example may result in a pair of identified BCG and PPG markers. A time difference between the pair of the identified BCG marker and PPG marker may be computed. This example may be repeated for other cardiac cycles. This may result in multiple time differences of the respective pairs of BCG and PPG markers. The temporal offset may, for example, be the average of the time differences.

In an alternative example, the BCG waveforms of multiple cardiac cycles of the extracted BCG signal are averaged to obtain one averaged BCG waveform that represents one cardiac cycle, and the PPG waveforms of multiple cardiac cycles of the extracted PPG signal are averaged to obtain one averaged PPG waveform that represents one cardiac cycle. The BCG marker may be identified (e.g., as any peak point) in the averaged BCG waveform and the PPG marker may be identified (e.g., as any zero-crossing point) in the averaged PPG waveform. The time difference between the identified pair of BCG and PPG markers may be the temporal offset. This example may have the advantage that the marker positions are better determined because the signal is less noisy after the average waveform determination.

The example offset determination techniques may be performed as part of the method for determining the trigger time i.e., using the BCG and PPG signals obtained from the (currently) received video frames. Alternatively, the example offset determination techniques may be performed as part of a calibration process which may be done before the present method of determining the trigger time is executed e.g., by using BCG and PPG signals which are previously obtained from the subject.

According to one example offset determination technique, the temporal offset may be estimated or predicted estimate from population statistics. This may enable to obtain the trigger time using the selected PPG marker only and by deploying coarse offset estimate from population statistics. In this case, the trigger time may, for example, be obtained as the result of shifting the selected PPG marker by the temporal offset.

Hence, the present subject matter may provide accurate triggering of medical data acquisition. The medical data acquisition may comprise MRI data acquisition or CT data acquisition. The present subject matter may further improve the accuracy of the acquisition triggering by using improved techniques to extract and separate the BCG signal from the PPG signal using the video frames. Embodiments may enable the extraction of information in either stream combined with mutual information cross-over of specific signal features.

The video frame may be a digital image. The image may have a spatial resolution. The spatial resolution may refer to the number of pixels composing the image. The term "region" as used herein may refer to a group of adjacent pixels. The present subject matter may provide in the video frames first regions which are candidate sources of PPG information. The first regions may, for example, be identified in each frame of the received video frames. For example, the first regions may be predefined. In this case, their identification may be performed by mapping their definitions to the frames. In another example, the first regions may be defined (dynamically) during the identification step. For example, a number $M$ of frames may be received. Each $i$-th frame $F_i$ of the received $M$ frames, may comprise first regions

$$PPG_1^{F_i} \ldots PPG_{Ni}^{F_i}$$
, where the subscript $Ni$ is the number of first regions in the frame $F_i$, where the index $i$ refers to the $i$-th frame and which is a number between 1 and the total number $M$ of received frames. The maximum number of first regions may be referred to as $N_{PPG} = \max \{Ni\}_{1 \leq i \leq M}$. For example, each frame of the received video frames may comprise the same number of first regions e.g., $Ni = Nj = N_{PPG}$, $\forall i \neq j$. Alternatively, a subset of frames of the received frames may comprise a number of first regions which is smaller than the maximum number of first regions $N_{PPG}$.

The identified maximum number of first regions $N_{PPG}$ may be used to determine the PPG signal. In one example, the PPG signal may be determined by processing frame by frame as follows. The PPG signal may comprise one signal value $S_{PPG}^i$ per frame $F_i$, e.g., the PPG signal may comprise $M$ signal values: $S_{PPG}^1, \ldots S_{PPG}^M$. The individual signal values $S_{PPG}^i$ may be obtained using different advantageous techniques. In one example, the signal value $S_{PPG}^i$ for a given frame $F_i$ may be obtained by summing all pixel values of the first regions $PPG_1^{F_i} \ldots PPG_{Ni}^{F_i}$ in the frame $F_i$ and dividing the sum by the number of pixels in the first regions $PPG_1^{F_i} \ldots PPG_{Ni}^{F_i}$ or by the number $Ni$ of first regions, e.g., $S_{PPG}^i = \dfrac{\sum_{k=1}^{Ni} S_{PPG_k}^{F_i}}{Ni}$, where $S_{PPG_k}^{F_i}$ is the average or weighted average of pixels values in the $k$-th first region $PPG_k^{F_i}$ or $S_{PPG}^i = \dfrac{\sum_{k=1}^{Ni} S_{PPG_k}^{F_i}}{npixels_i}$, $S_{PPG_k}^{F_i}$, is the sum of pixels values in the $k$-th first region $PPG_k^{F_i}$ and $npixels_i$ is the number of pixels in the first regions of the $i$-th frame. The PPG signal $S_{PPG}$ may thus be defined as follows:

$$S_{PPG} = \{S_{PPG}^1, S_{PPG}^2 \ldots, S_{PPG}^M\}.$$

The present subject matter may further provide in the received video frames second regions which are candidate sources of BCG information. The second regions may, for example, be identified in each frame of received video frames. For example, each $i$-th frame $F_i$ of the received M frames, may comprise second regions $BGG_1^{F_i} \ldots BCG_{Li}^{F_i}$, where the subscript $Li$ is the number of second regions in the frame $F_i$, where the index $i$ refers to the $i$-th frame and which is a number between 1 and the total number $M$ of received frames. The maximum number of second regions may be referred to as $N_{BCG} = \max \{Li\}_{1 \leq i \leq M}$. For example, each frame of the received video frames may comprise the same number of second regions e.g., $Li = Lj = N_{BCG}$, $\forall i \neq j$. Alternatively, a subset of frames of the received frames may comprise a number of second regions which is smaller than the maximum number of second regions $N_{BCG}$.

The identified maximum number of second regions $N_{BCG}$ may be used to determine $N_{BCG}$ candidate BCG signals, one per second region. The $N_{BCG}$ candidate BCG signals may be referred to as $S_{BCG_1}, S_{BCG_2} \ldots S_{BCG_{N_{BCG}}}$. In one example, each candidate BCG signal may be determined by processing frame by frame the received frames as follows. For each $k$-th second region $BCG_k$, and for each $i$-th frame $F_i$, one signal value $S_{BGG_k}^{F_i}$ may be obtained from the second region $BGG_k^{F_i}$ in the frame by averaging the pixel values in the second region $BGG_k^{F_i}$. Thus, the candidate BCG signal $S_{BCG_k}$ for the $k$-th second region $BCG_k$ may comprise the values: $S_{BGG_k}^{F_1}, S_{BGG_k}^{F_2} \ldots S_{BGG_k}^{F_M}$. In case a frame does not have the $k$-th second region $BCG_k$, the associated signal value may be set to zero or may be interpolated from the remaining signal values. Thus, the $N_{BCG}$ candidate BCG signals may, for example, be defined as follows: $S_{BCG_1} = \{S_{BGG_1}^{F_1}, S_{BGG_1}^{F_2} \ldots, S_{BGG_1}^{F_M}\}, S_{BCG_2} = \{S_{BGG_2}^{F_1}, S_{BGG_2}^{F_2} \ldots, S_{BGG_2}^{F_M}\}, \ldots, S_{BCG_{N_{BCG}}} = \{S_{BGG_{N_{BCG}}}^{F_1}, S_{BGG_{N_{BCG}}}^{F_2} \ldots, S_{BGG_{N_{BCG}}}^{F_M}\}.$

The similarity or dissimilarity of the set of candidate BCG signals $S_{BCG_1}$ $S_{BCG_2}, \ldots, S_{BCG_{N_{BCG}}}$ with the PPG signal $S_{PPG}$ may be determined. This may result in a number $N_{BCG}$ of similarity values $SIM_1, \ldots, SIM_{N_{BCG}}$. For example, the similarity values may be defined as follows: $SIM_1 =$ similarity($S_{PPG}$, $S_{BCG1}$), $SIM_2 = \text{similarity}(S_{PPG}, S_{BCG_2}), \ldots, SIM_{N_{BCG}} = \text{similarity}(S_{PPG}, S_{BCG_{N_{BCG}}})$ , where similarity(.) is a similarity or dissimilarity function.

Using the similarity values, a subset of or all of the second regions may be selected. For example, the similarity values may be compared against a threshold. If a similarity value exceeds the threshold, the corresponding second region may be selected. The selected regions may be referred to as $BCG_1 \ldots BCG_{N_{BCG}^{sel}}$ , where $N_{BCG}^{sel}$ is the number of selected second regions which may be smaller than or equal to the number of identified second regions $N_{BCG}$, $N_{BCG}^{sel} \leq N_{BCG}$.

A BCG signal may be built using the selected second regions. The BCG signal $S_{BCG}$ may, for example, be obtained by combining the candidate BCG signals previously determined: $S_{BCG_1} = \{S_{BGG_1^{F_1}}, S_{BGG_1^{F_2}} \ldots, S_{BGG_1^{F_M}}\}, S_{BCG_2} = \{S_{BGG_2^{F_1}}, S_{BGG_2^{F_2}} \ldots, S_{BGG_2^{F_M}}\}, \ldots, S_{BCG_{N_{BCG}^{sel}}} = \{S_{BGG_{N_{BCG}^{sel}}^{F_1}}, S_{BGG_{N_{BCG}^{sel}}^{F_2}} \ldots, S_{BGG_{N_{BCG}^{sel}}^{F_M}}\}$ . For example, the BCG signal may be the spatial average of the candidate BCG signals of the selected second regions: $S_{BCG} = \text{average}(S_{BCG_1}, S_{BCG_2}, \ldots, S_{BCG_{N_{BCG}^{sel}}})$ . In the following, another alternative method based on clustering may be used to determine the BCG signal $S_{BCG}$.

The present subject matter may thus use a remote camera-based PPG and BCG measurement to identify BCG and PPG regions. Although the BCG signals are small, the corresponding regions may be correctly identified according to the present subject matter. This correct region identification may lead to accurate BCG signals. In addition, the BCG signal may be used in combination with the PPG signal to provide accurate identification of the temporal offset. The BCG signals may be used to do triggering and to calibrate (personalize) the PPG triggering by measuring the temporal offset as well. The present subject matter may enable accurate and continuous monitoring of the state of the heart as required in for example MRI and CT triggering applications.

The present subject matter may achieve two objectives at once: a hassle-free measurement (for patient and personnel, e.g., simplifying the workflow) and a proper cardiac gating which may be characterized by a strong coupling to specific features in the ECG cycle, in particular to the R-peak, useable in both prospective and retrospective scanning modes.

An alternative determination of the PPG signal $S_{PPG}$ may be provided. According to one example, the PPG signal $S_{PPG}$ may be determined as follows. The identified maximum number $N_{PPG}$ of first regions may be used to determine $N_{PPG}$ candidate PPG signals, one per first region. The $N_{PPG}$ candidate PPG signals may be referred to as $S_{PPG_1}, S_{PPG_2}, \ldots S_{PPG_{N_{PPG}}}$ respectively. In one example, each candidate PPG signal may be determined by processing frame by frame the received frames as follows. For each $k$-th first region $PPG_k$, and for each $i$-th frame $F_i$, one signal value $S_{PPG_k^{F_i}}$ . may be obtained from the first region $PPG_k^{F_i}$ in the frame $F_i$ by averaging or weight averaging the pixel values in the first region $PPG_k^{F_i}$ . Thus, the candidate PPG signal $S_{PPG_k}$ for the $k$-th first region $PPG_k$ may comprise the $M$ values: $S_{PPG_k^{F_1}}, S_{PPG_k^{F_2}} \ldots S_{PPG_k^{F_M}}$ , one per frame. In case a frame does not have the $k$-th first region $PPG_k$, the associated signal value may be set to zero or may be estimated e.g., using interpolation from the remaining signal values. Thus, the $N_{PPG}$ candidate PPG signals may, for example, be defined as follows:

$$S_{PPG_1} = \{S_{PPG_1}^{F_1}, S_{PPG_1}^{F_2} \ldots, S_{PPG_1}^{F_M}\}, S_{PPG_2} = \{S_{PPG_2}^{F_1}, S_{PPG_2}^{F_2} \ldots, S_{PPG_2}^{F_M}\}, \ldots, S_{PPG_{N_{PPG}}} = \{S_{PPG_{N_{PPG}}}^{F_1}$$

$$S_{PPG_{N_{PPG}}}^{F_2} \ldots, S_{PPG_{N_{PPG}}}^{F_M}\}$$
, . The candidate PPG signals and the candidate BCG signals may be clustered to obtain one cluster that represents BCG signals and another cluster that represents PPG signals. The clustering may further result in a third cluster which represents other signals different from both the BCG and PPG signals. That is, the $N_{PPG}$ + $N_{BCG}$ signals $S_{BCG_1}, S_{BCG_2} \ldots S_{BCG_{N_{BCG}}}$ and $S_{PPG1}, S_{PPG_2} \ldots S_{PPG_{N_{PPG}}}$ may be clustered. The signals of the BCG cluster may be combined for obtaining the BCG signal $S_{BCG}$. The signals of the PPG cluster may be combined for obtaining the PPG signal $S_{PPG}$. The combination may, for example, be performed by averaging.

This example may be advantageous because the clustering may enable to (re-)cluster the candidate BCG and PPG signals to correct for potential improper identifications of the first and second regions. Advantageously, the clustering operation may be performed with the aim to remove BCG information which erroneously was identified and used in the construction of the PPG signal. This example may thus further improve the accuracy of the PPT detection.

According to one example, the clustering comprises: evaluating a signal feature for the candidate PPG and BCG signals, and building the clusters using the evaluated signal feature, wherein the signal feature comprises at least one of: the waveform of the signal, relative phase of pairs of signals, and location of the first and second regions.

This example may enable a flexible and controllable clustering. And the combination of more than one signal feature may improve the clustering. The clustering may, for example, be performed using more than one signal feature as follows. Location information of regions may be used to identify initial BCG and PPG clusters e.g., the position of certain regions may identify them as either likely dominated by BCG signal or by PPG signal. In addition, the remaining regions may be clustered as belonging to either class based on highest similarity of waveform and optionally further based on their relative phase. This may be advantageous as PPG waveforms and BCG waveforms are different.

According to one example, the PPG signal $S_{PPG}$ may be determined as follow. A subset of the set of candidate PPG signals $S_{PPG_1}, S_{PPG_2} \ldots S_{PPG_{N_{PPG}}}$ may be selected based on their similarity with the BCG signal $S_{BCG}$. This may be equivalent to select subset of first regions from the first regions since the candidate PPG signals are associated with the first regions respectively. A number $N_{PPG}$ similarity values $sim_1, \ldots, sim_{N_{PPG}}$ may, for example, be defined. For example, the similarity values may be defined as follows: $sim_1 = similarity(S_{BCG}, S_{PGG_1})$, $sim_2 = similarity(S_{BCG}, S_{PGG_2})$, ..., $sim_{N_{PPG}} = similarity(S_{BCG}, S_{PGG_{N_{PPG}}})$ . Using the similarity values, a subset of or all of the first regions may be selected e.g., the similarity values may be compared against a threshold. If a similarity value exceeds the threshold, the corresponding first region may be selected. The selected first regions may be referred to as $PPG_1 \ldots PGG_{N_{PPG}^{sel}}$ , where $N_{PPG}^{sel}$ is the number of selected first regions which may be smaller than or equal to the number of identified first regions $N_{PG}$, $N_{PPG}^{sel} \leq N_{PPG}$ . The PPG signal may be obtained by combining the selected candidate PPG signals. For example, the PPG signal may be the average of the candidate PPG signals of the selected first regions: $S_{PPG} = \text{average}(S_{PPG_1}, S_{PPG_2}, \ldots, S_{PPG_{N_{PPG}^{sel}}})$ . Alternatively, the PPG signal may be the average of the candidate PPG signals of the selected first regions: $S_{PPG} = \text{average}(S_{PPG_1}, S_{PPG_2}, \ldots, S_{PPG_{N_{PPG}^{sel}}})$ .

This example may be advantageous because the similarity measure may enable to refine the list of candidate PPG signals to correct for potential improper identifications of the first regions.

According to one example, the received video frames are acquired during a breath-hold period of the subject.

This example may be advantageous because the PPG and in particular BCG signals are most pronounced during breath-holds. Therefore, these periods may enable an efficient identification of BCG regions and PPG regions.

According to one example, each frame of the frames comprises the first regions, wherein determining of the first PPG signal using the first regions comprises: combining pixel values in the first regions of each frame for obtaining one PPG signal value per frame of the frames.

According to one example, each frame of the frames comprises the selected second regions, wherein determining the BCG signal using the selected second regions comprises: combining pixel values in the selected second regions of each frame for obtaining one BCG signal value per frame of the frames.

According to one example, comparing the set of candidate BCG signals with the first PPG signal comprises correlating the set of candidate BCG signals with the first PPG signal, wherein the similarity values are the correlation values respectively. The correlation may provide an accurate similarity metric in particular as the BCG and PPG signals are time domain signals. Alternatively, comparing the set of candidate BCG signals with the first PPG signal comprises determining the similarity of the set of candidate BCG signals with the first PPG signal. However, other similarity metrics may be used. One example may be to add a term to the correlation function that enhances the correlation if the relative phase of PPG and BCG is in the expected range derived from a typical PTT. The assumed or typical PTT range may be derived from prior information such as patient size and weight. For example, the correlation function may be defined as $\max(\mathrm{xcorr}(S_{BBG}, S_{BCG}))$, where xcorr is the cross-correlation of both signals. The location of this maximum in the cross-correlation defines the relative phase between both signals. The enhanced correlation function may be defined as $\max(\mathrm{xcorr}(S_{PPG}, S_{BCG})[\mathrm{tau} - r, \mathrm{tau} + r])$ where tau is the expective relative phase and r the radius of search (parameter). A third correlation function may be defined as: $\exp(-|t - \mathrm{tau}| / C)\max(\mathrm{xcorr}(S_{BBG}, S_{BCG}))$, where t is the measured relative phase, tau is the expected relative phase and C a decay parameter.

According to one example, the medical system further comprises a magnetic resonance imaging system, wherein the memory further contains pulse sequence commands configured to control the magnetic resonance imaging system to acquire k-space data according to the magnetic resonance imaging protocol, wherein execution of the machine executable instructions further causes the computational system to use the PTT to acquire k-space data from the subject by controlling the magnetic resonance imaging system.

In one example, the PTT values may be used to derive the position of the R-peak of the ECG signal. The R-peak may be used as a trigger to acquire the MRI data from the subject.

According to one example, the first regions represent skin areas of the subject and the second regions representing non-skin areas of the subject. Skin areas may be areas from which we can measure a PPG. Skin areas may refer to pulsatile areas of the subject where bare skin is visible. Non-skin areas may be defined as pulsatile areas of the subject where no bare skin is visible. The non-skin area of the subject may, for example, be the chest or shoulders of the subject.

According to one example, the first regions may be identified by: identifying a working area of skin pixels; creating candidate regions in the working area; correcting signals in each candidate region from external interferences and motion artefacts; selecting the first regions from the candidate regions using the corrected signals.

According to one example, the identification of the working area comprises detecting a face area of the subject.

In another aspect the invention relates to a computer program comprising machine executable instructions for execution by a computational system; wherein execution of the machine executable instructions causes the computational system to: receive video frames of a video which have been acquired from a subject; determine a photoplethysmogram, PPG, signal and a ballistocardiogram, BCG, signal from the video frames; identify first markers in the PPG signal; identify second markers in the BCG signal; define a trigger time for acquiring medical data from the subject using a selected first marker and/or selected second marker.

In another aspect the invention relates to a method comprising: receiving video frames of a video which have been acquired from a subject; determining a photoplethysmogram, PPG, signal and a ballistocardiogram, BCG, signal from the video frames; identifying first markers in the PPG signal; identifying second markers in the BCG signal; defining a trigger time for acquiring medical data from the subject using a selected first marker and/or selected second marker.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid-state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. 'Computer storage' or 'storage' is a further example of a computer-readable storage medium. Computer storage is any non-volatile computer-readable storage medium. In some embodiments computer storage may also be computer memory or vice versa.

A 'computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen, Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

K-space data is defined herein as being the recorded measurements of radio frequency signals emitted by atomic spins using the antenna of a Magnetic resonance apparatus during a magnetic resonance imaging scan. Magnetic resonance data is an example of tomographic medical image data.

A Magnetic Resonance image or MR image is defined herein as being the reconstructed two- or three-dimensional visualization of anatomic data contained within the k-space data. This visualization can be performed using a computer.

BRIEF DESCRIPTION OF THE DRAWINGS

In the following preferred embodiments of the invention will be described, by way of example only, and with reference to the drawings in which:

Fig. 1 illustrates an example of a medical system;
Fig. 2 shows a flowchart of a method of determining a trigger time for acquisition of medical data from a subject;
Fig. 3 shows a flowchart of a method of determining the BCG signal and PPG signal from video frames;
Fig. 4 depicts a diagram illustrating a method for identification of regions in the frames;
Fig. 5 illustrates an example of a medical system;
Fig. 6 shows a flowchart of a method of determining PPG signals;
Fig. 7 shows a flowchart of a method of determining the temporal offset;
Fig. 8 depicts a diagram of a system of determining PPG and BCG signals;
Fig. 9 depicts a diagram of a system of determining PPG and BCG signals;
Fig. 10 depicts a diagram of a system of determining PPG and BCG signals;
Fig. 11 shows a correlation coefficient heat map.

DETAILED DESCRIPTION OF EMBODIMENTS

Like numbered elements in these figures are either equivalent elements or perform the same function. Elements which have been discussed previously will not necessarily be discussed in later figures if the function is equivalent.

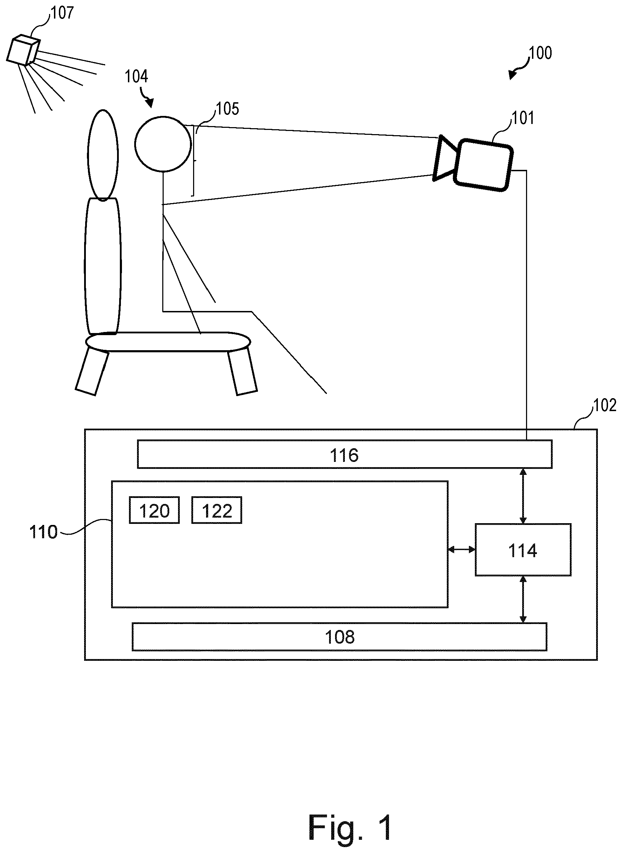

Fig. 1 shows an example of a medical system 100. There is a subject 104 reposing on a seat. A camera 101 is focused on an imaging zone 105 of the subject 104. The camera 101 is connected to a hardware interface 116 of a computer system 102. The camera 101 may be configured to acquire video data from the imaging zone 105, to store the video data and transmit the video data to the computer system 102. The video data may, for example, be transmitted as a stream. For example, the computer system 102 may control the acquisition of the video data by the camera 101. A light source 107 is provided in order to illuminate the subject 104 and in particular the imaging zone 105.

The computer system 102 is provided with a processor 114. The computer system 102 is intended to represent one or more computers or computer systems. The processor 114 is intended to represent one or more computational systems or computational cores. The computer system 102 is further shown as containing the hardware interface 116 which is connected to the processor 114. If other components of the medical system 100 are present or included such as a magnetic resonance imaging system, then the hardware interface 116 could be used to exchange data and commands with these other components. The computer system 102 is further shown as comprising an optional user interface 108 which may provide various means for relaying data and receiving data and commands from an operator.

The computer system 102 is further shown as comprising a memory 110 that is connected to the processor 114. The memory 110 is intended to represent various types of memory which may be accessible to the processor 114. The memory 110 is shown as containing machine-executable instructions 120. The machine-executable instructions 120 are instructions which enable the processor 114 to perform various control, data processing, and image processing tasks. The memory 110 is further shown as containing videos 122 acquired by the camera 101.

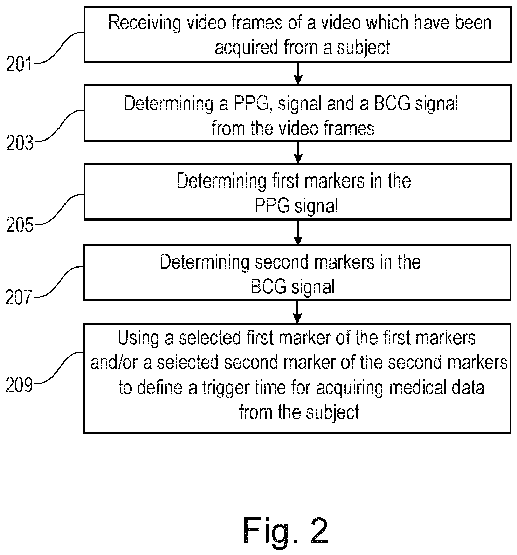

The present subject matter may use the medical system 100 to perform a method such as the method of Fig. 2 in order to determine PTT of the subject 104.

Fig. 2 is a flowchart of a method for determining a trigger time for acquisition of medical data in accordance with an example of the present subject matter. For the purpose of explanation, the method described in Fig. 2 may be implemented in the system illustrated in Fig. 1, but is not limited to this implementation. The method may, for example, be performed by the medical system 100 or by another system that is configured to connect to the medical system 100.

Video frames of a video which have been acquired from a subject 104 may be received in step 201. A PPG signal and a BCG signal may be derived or determined in step 203 from the received video frames. First markers may be determined or identified in step 205 in the PPG signal. Second markers may be determined or identified in step 207 in the BCG signal. A selected first marker of the first markers and/or a selected second marker of the second markers may be used in step 209 to define a trigger time for acquiring medical data from the subject.

In one example implementation, named real-time implementation, the method of Fig. 2 may be performed for acquisition of MRI data from the subject 104 using the determined trigger time. In one example implementation, named offline implementation, the method of Fig. 2 may be performed for measuring vital sign information of the subject 104.

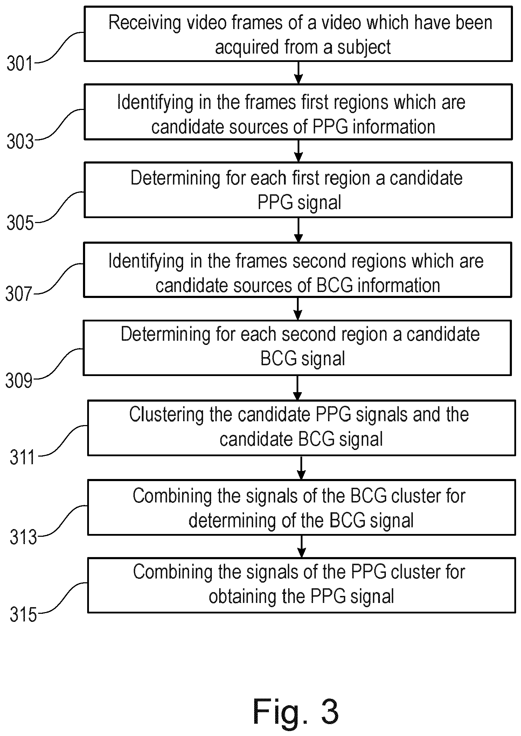

Fig. 3 is a flowchart of a method for determining a PPG signal and BCG signal in accordance with an example of the present subject matter. For the purpose of explanation, the method described in Fig. 3 may be implemented in the system illustrated in Fig. 1, but is not limited to this implementation. The method may, for example, be performed by the medical system 100 or by another system that is configured to connect to the medical system 100. The method of Fig. 3 may provide an example implementation for obtaining the PPG signal and the BCG signal in Fig. 2.

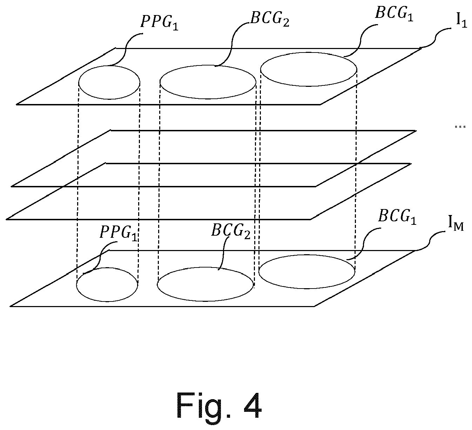

Video frames of a video which have been acquired from a subject 104 may be received in step 301. First regions which are candidate sources of PPG information may be identified in the frames in step 303. A candidate PPG signal may be determined in step 305 for each first region of the first regions. This may result in a set of candidate PPG signals. Second regions which are candidate sources of BCG information may be identified in the frames in step 307. The second regions are provided such that they are different from the first regions. Fig. 4 illustrate an example distribution of the identified first and second regions. It may be determined in step 309 for each second region a candidate BCG signal. This may result in a set of candidate BCG signals.

A clustering of the candidate PPG signals and the candidate BCG signals may be performed in step 311 in order to obtain a cluster (BCG cluster) of signals representing BCG and another cluster (PPG cluster) of signals representing PPG. This may further result in a third cluster which represent signals different from both the BCG signals and PPG signals.

The signals of the BCG cluster may be combined in step 313 for obtaining of the BCG signal. The signals of the PPG cluster may be combined in step 315 for obtaining the PPG signal. The combination may, for example, be the average.

Fig. 4 depicts a series of $M$ frames (or images) $I_1...I_M$ of a received video. For example, the series of frames $I_1...I_M$ may be the frames received in step 201. For exemplification purpose, Fig. 4 depicts a first region (PPG region) and two second regions (BCG regions) $PPG_1$, $BCG_1$ and $BCG_2$. As indicated in Fig. 4, the regions reappear in each frame of the frames. Each region has its own set of pixels.

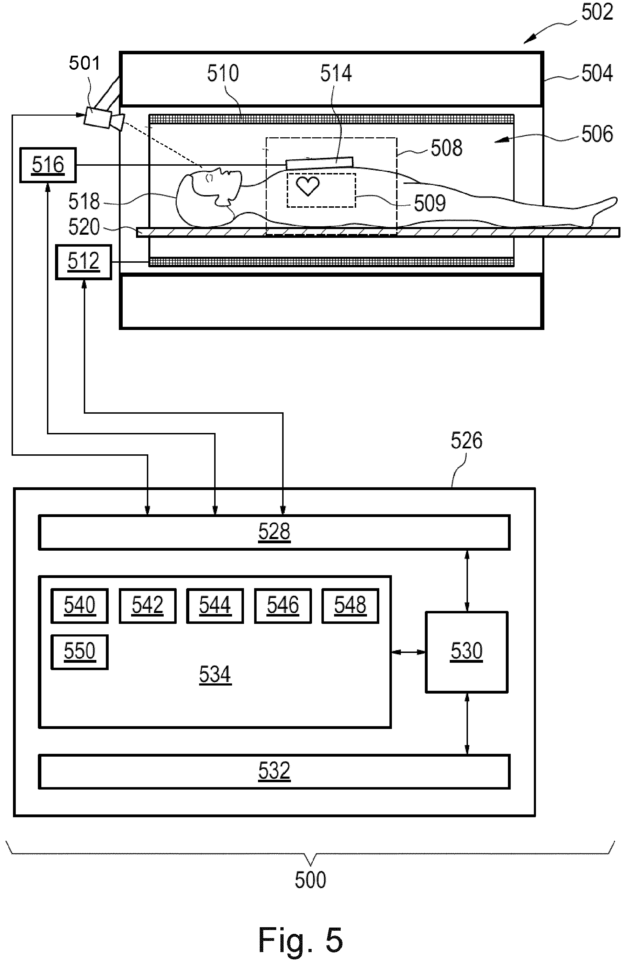

Fig. 5 shows a further example of a medical instrument 500. In this example the medical instrument 500 comprises a magnetic resonance imaging system 502. The magnetic resonance imaging system 502 comprises a magnet 504. The magnet 504 is a superconducting cylindrical type magnet with a bore 506 through it. The use of different types of magnets is also possible; for instance, it is also possible to use both a split cylindrical magnet and a so-called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils. Within the bore 506 of the cylindrical magnet 504 there is an imaging volume 508 where the magnetic field is strong and uniform enough to perform magnetic resonance imaging. A region of interest 509 is shown within the imaging volume 508. A subject 518 is shown as being supported by a subject support 520 such that at least a portion of the subject 518 is within the imaging volume 508 and the region of interest 509.

Within the bore 506 of the magnet there is also a set of magnetic field gradient coils 510 which is used for acquisition of magnetic resonance data to spatially encode magnetic spins within the imaging volume 508 of the magnet 504. The magnetic field gradient coils 510 connected to a magnetic field gradient coil power supply 512. The magnetic field gradient coils 510 are intended to be representative. Typically, magnetic field gradient coils 510 contain three separate sets of coils for spatially encoding in three orthogonal spatial directions. A magnetic field gradient power supply supplies current to the magnetic field gradient coils. The current supplied to the magnetic field gradient coils 510 is controlled as a function of time and may be ramped or pulsed.

Adjacent to the imaging volume 508 is a radio-frequency coil or surface coil 514 for manipulating the orientations of magnetic spins within the imaging volume 508 and for receiving radio transmissions from spins also within the imaging volume 508. The radio frequency antenna may contain multiple coil elements. The radio frequency antenna may also be referred to as a channel or antenna. The radio-frequency coil 514 is connected to a radio frequency transceiver 516. The radio-frequency coil 514 and radio frequency transceiver 516 may be replaced by separate transmit and receive coils and a separate transmitter and receiver. It is understood that the radio-frequency coil 514 and the radio frequency transceiver 516 are representative. The radio-frequency coil 514 is intended to also represent a dedicated transmit antenna and a dedicated receive antenna. Likewise, the transceiver 516 may also represent a separate transmitter and receivers. The radio-frequency coil 514 may also have multiple receive/transmit elements and the radio frequency transceiver 516 may have multiple receive/transmit channels. For example, if a parallel imaging technique such as SENSE is performed, the radio-frequency coil 514 will have multiple coil elements.

In this example a camera 501 can be seen as being mounted on the flange of the magnet 504. The face of the subject is imaged by the camera 501.

The transceiver 516, the gradient controller 512, and camera 501 are shown as being connected to a hardware interface 528 of a computer system 526. The computer system further comprises a processor 530 that is in communication with the hardware system 528, a memory 534, and a user interface 532. The memory 534 may be any combination of memory which is accessible to the processor 530. This may include such things as main memory, cached memory, and also non-volatile memory such as flash RAM, hard drives, or other storage devices. In some examples the memory 530 may be considered to be a non-transitory computer-readable medium.

The computer memory 534 is shown as containing machine-executable instructions 540. The machine-executable instructions contain commands or instructions which enable the processor 530 to control the operation and function of the magnetic resonance imaging system 502. The computer memory 534 is shown as further containing pulse sequence commands 542. The pulse sequence commands 542 are either instructions or data which may be converted into instructions which enable the processor 530 to control the magnetic resonance imaging system 502 to acquire magnetic resonance data.

The computer memory 534 is shown as containing video frames 544 acquired with the camera 501. The various frames in the video frames 544 may be used to derive the trigger time 546 for acquisition of data from the subject 518. For example, the method illustrated in Fig. 2 or Fig. 3, could be performed repeatedly to generate the trigger time 546 which can be used to trigger the acquisition of magnetic resonance data 548 using the pulse sequence commands 542. The magnetic resonance data 548 can then be reconstructed into a magnetic resonance image 550.

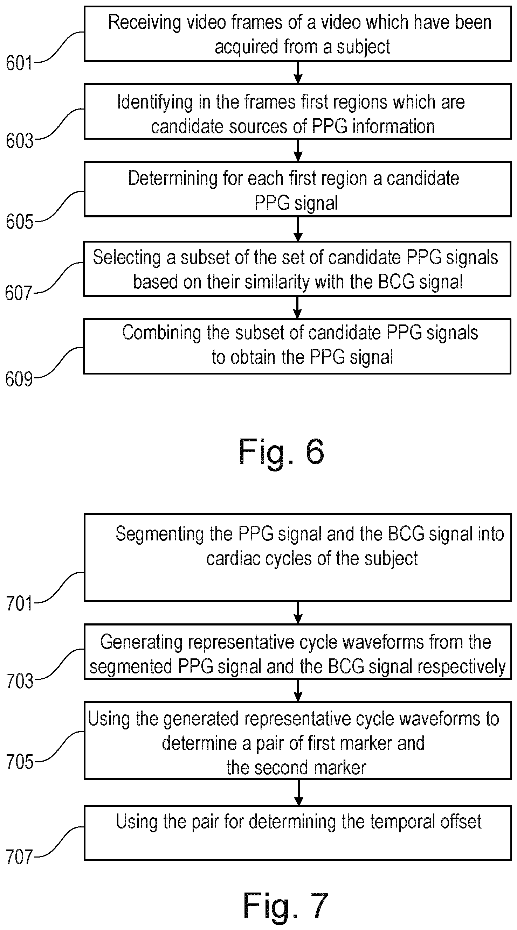

Fig. 6 is a flowchart of a method for determining a PPG signal in accordance with an example of the present subject matter. For the purpose of explanation, the method described in Fig. 6 may be implemented in the system illustrated in Fig. 1, but is not limited to this implementation. The method may, for example, be performed by the medical system 100 or by another system that is configured to connect to the medical system 100. The method of Fig. 6 may provide an example implementation for obtaining the PPG signal in Fig. 2.

Video frames of a video which have been acquired from a subject 104 may be received in step 601. First regions which are candidate sources of PPG information may be identified in the frames in step 603. It may be determined in step 605 for each first region of the identified first regions a candidate PPG signal. This may result in a set of candidate PPG signals. A subset of the set of candidate PPG signals may be selected in step 607 based on their similarity with the BCG signal. The subset of candidate PPG signals may be combined in step 609 to obtain the second PPG signal. The combination may, for example, be the average.

Fig. 7 is a flowchart of a method for determining the temporal offset from a BCG signal and PCG signal in accordance with an example of the present subject matter. For the purpose of explanation, the method described in Fig.

7 may be implemented in the system illustrated in Fig. 1, but is not limited to this implementation. The method may, for example, be performed by the medical system 100 or by another system that is configured to connect to the medical system 100.

The PPG signal and the BCG signal may be segmented in step 701 into cardiac cycles of the subject. Representative cycle waveforms may be generated in step 703 from the segmented PPG signal and the BCG signal respectively. The generated representative cycle waveforms may be used to determine in step 705 a pair of first marker and the second marker. The pair of the first marker and the second marker may be used in step 707 for determining the temporal offset.

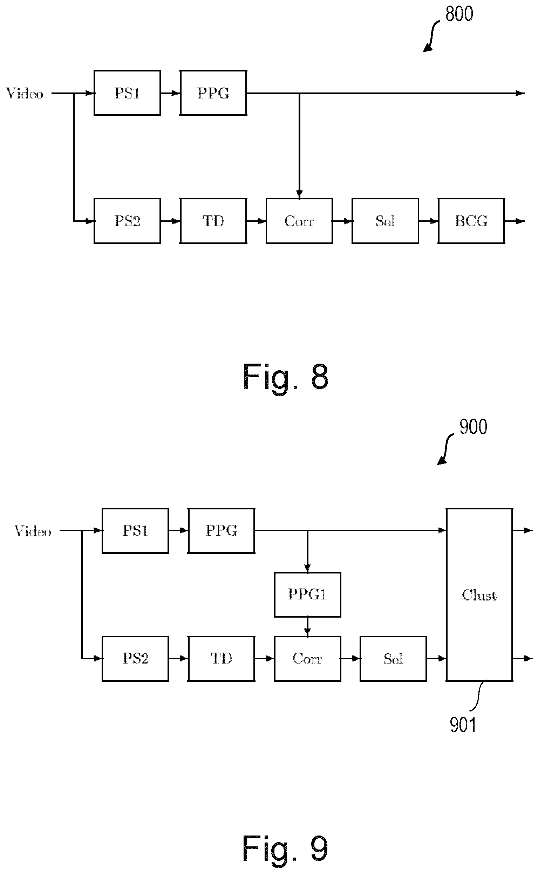

In Fig. 8, a system 800 is shown where the PPG signal guides the BCG extraction. The system 800 is shown as containing units PS1, PS2, TD, Corr, Sel, PPG and BCG. The incoming video is input to two patch creation units. The first patch creation unit PS1 has automatic PPG area selection, and from that creates an overall PPG signal in unit PPG. The second patch creation unit PS2 has multiple patches potentially holding BCG content but not coinciding with the PPG patches. For these patches, a time domain signal is created as the average over the patch in unit TD. These time domain signals are correlated with the PPG signal. Next, BCG patches are selected in unit Sel as those having a high correlation and a sensible relative phase. The signals from the BCG patches are combined into a BCG signal in unit BCG. The exact PTT can finally be derived from the maximum of the final BCG and PTT signals.

In Fig. 9, a system 900 is shown involving clustering. Units PS1, PS2, TD, Corr and Sel are as described with reference to Fig. 8. However, the unit PPG in Fig. 9 creates PPG signals per patch, and an additional unit PPG1 creates an initial PPG signal used for correlation from the ensemble of PPG signals per patch; this could for instance be a simple averaging operation. The PPG signal of the patches in the upper branch and the BCG signals from the selected BCG patches in the lower branch are input to a clustering algorithm 901. This clustering algorithm 901 (re-)clusters the time domain signals of BCG and PPG signals to correct for potential improper choices in units PS1 and PS2. The output of the cluster block consists of the PPG and BCG signals representing both clusters.

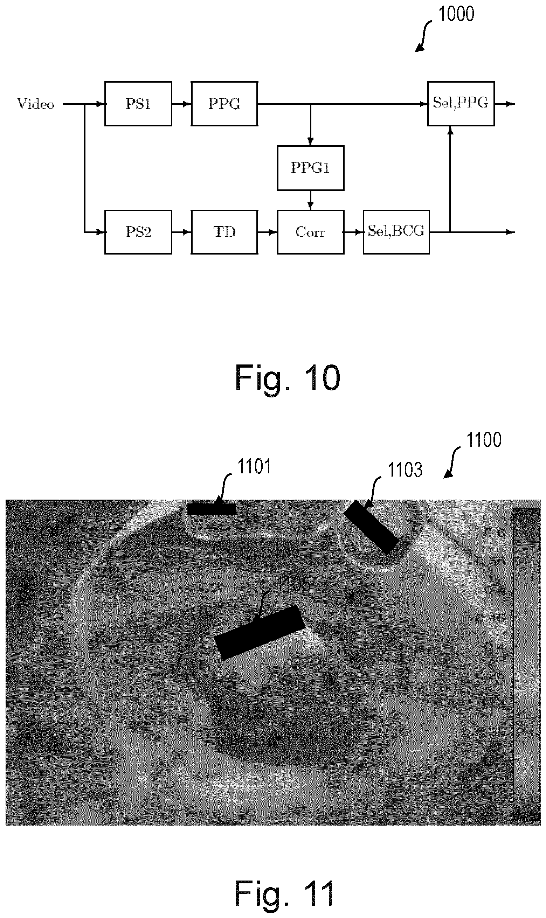

In Fig. 10, a system 1000 is shown involving correcting for erroneously selected patches in unit PS1. The Units PS1, PS2, TD, Corr, PPG and PPG1 are as described in Fig. 9. The unit Sel,BCG in the lower branch selects BCG patches and creates the BCG signal. The BCG signal is compared to the signals of the patches that were initially selected in the upper branch as PPG patches. Patches holding signals resembling the BCG signal too closely are removed and, from the remaining patches, the PPG signal is constructed in unit Sel,PPG.

Thus, a PPG signal may be constructed from the video sequence. A first instance of the PPG signal is made. Next, the time domain signals from average over patches of the image (or selected parts thereof, e.g., by excluding all parts earlier identified as holding PPG information) are correlated with the constructed PPG signal. Patches that contain BCG information are thus identified by the correlation. This is shown in Fig. 11, where an image 1100 is shown with a superimposed heat map of the correlation coefficients of the PPG signal and the time domain signals. The vertical bar indicates normalized values of the amplitude of the signal. The vertical bar indicates that the correlation areas are on the face and also on the chest. Patches 1101, 1103 and 1105 may be blacked-out regions which are added for restraining the identification of the subject.

## Claims

1. A system comprising: a memory storing machine executable instructions; and a computational system, wherein execution of the machine executable instructions causes the computational system to:

   receive video frames of a video which have been acquired from a subject;
   determine a photoplethysmogram, PPG, signal and a ballistocardiogram, BCG, signal from the video frames;
   determine first markers in the PPG signal;
   determine second markers in the BCG signal;
   use a selected first marker of the first markers and/or a selected second marker of the second markers to define a trigger time for acquiring medical data from the subject.

2. The system of claim 1, wherein execution of the machine executable instructions causes the computational system to define the trigger time using the selected first marker and further using a temporal offset between the PPG signal and the BCG signal.

3. The system of claim 2, wherein the temporal offset is determined by: comparing the first markers and second markers for identifying unique pairs of first marker and second marker, such that each pair of first marker and second marker covers a time interval smaller than one cardiac cycle of the subject, wherein the temporal offset is determined using the time intervals.

4. The system of any of the preceding claim 2, wherein execution of the machine executable instructions causes the computational system to determine the temporal offset by:

> segmenting the PPG signal and the BCG signal into cardiac cycles of the subject;
> generating representative cycle waveforms from the segmented PPG signal and the BCG signal respectively;
> using the generated representative cycle waveforms to determine a pair of first marker and the second marker;
> using the pair for determining the temporal offset.

5. The system of any of the preceding claims, wherein execution of the machine executable instructions causes the computational system to:

> identify in the frames first regions which are candidate sources of PPG information;
> determine for each first region a candidate PPG signal, resulting in a set of candidate PPG signals;
> identify in the frames second regions which are candidate sources of BCG information, the second regions being different from the first regions;
> determine for each second region a candidate BCG signal, resulting in a set of candidate BCG signals;
> cluster the candidate PPG signals and the candidate BCG signals, resulting in at least a cluster of signals representing BCG and another cluster of signals representing PPG;
> combine the signals of the BCG cluster for performing the determining of the BCG signal;
> combine the signals of the PPG cluster for the determining of the PPG signal.

6. The system of any of the preceding claims 1 to 4, wherein execution of the machine executable instructions causes the computational system to:

> identify in the frames first regions which are candidate sources of PPG information;
> determine for each first region a candidate PPG signal, resulting in a set of candidate PPG signals,
> selecting a subset of the set of candidate PPG signals based on their similarity with the BCG signal;
> combining the subset of candidate PPG signals to obtain the PPG signal.

7. The system of any of the preceding claims, wherein execution of the machine executable instructions causes the computational system to:

> identify in the frames second regions which are candidate sources of BCG information;
> determine for each second region a candidate BCG signal, resulting in a set of candidate BCG signals;
> compare the set of candidate BCG signals with the PPG signal, resulting in a set of similarity values;
> select second regions from the second regions which are associated with similarity values that exceed a threshold;
> perform the determination of the BCG signal using the selected second regions.

8. The system of any of the preceding claims, wherein execution of the machine executable instructions causes the computational system to acquire the video frames during a breath-hold period of the subject.

9. The system of any of the preceding claims, wherein execution of the machine executable instructions causes the computational system to:
identify in the frames first regions which are candidate sources of PPG information; wherein each frame of the frames comprises the first regions, wherein determining of the PPG signal is performed using the first regions by combining pixel values in the first regions of each frame for obtaining one PPG signal value per frame of the frames.

10. The system of any of the preceding claims, wherein execution of the machine executable instructions causes the computational system to:
identify in the frames second regions which are candidate sources of, BCG information; wherein each frame of the frames comprises the second regions, wherein determining the BCG signal is performed using the second regions by combining pixel values in the second regions of each frame for obtaining one BCG signal value per frame of the frames.

11. The system of any of the preceding claims, wherein the system further comprises a magnetic resonance imaging system, wherein the memory further contains pulse sequence commands configured to control the magnetic resonance imaging system to acquire k-space data according to the magnetic resonance imaging protocol, wherein execution of the machine executable instructions further causes the computational system to use the trigger time to acquire k-space data from the subject by controlling the magnetic resonance imaging system.

12. The system of any of the preceding claims 5 to 11, the first regions representing skin areas of the subject and the second regions representing non-skin areas of the subject.

13. The system of claim 12, the skin area comprising a face area of the subject, the non-skin area comprising a chest area or a shoulder area of the subject.

14. A computer program comprising machine executable instructions, wherein execution of the machine executable instructions causes a computational system to:

receive video frames of a video which have been acquired from a subject;
determine a photoplethysmogram, PPG, signal and a ballistocardiogram, BCG, signal from the video frames;
identify first markers in the PPG signal;
identify second markers in the BCG signal;
define a trigger time for acquiring medical data from the subject using a selected first marker and/or selected second marker.

15. A method comprising:

receiving video frames of a video which have been acquired from a subject;
determining a photoplethysmogram, PPG, signal and a ballistocardiogram, BCG, signal from the video frames;
identifying first markers in the PPG signal;
identifying second markers in the BCG signal;
defining a trigger time for acquiring medical data from the subject using a selected first marker and/or selected second marker.

Fig. 1

201 — Receiving video frames of a video which have been acquired from a subject

203 — Determining a PPG, signal and a BCG signal from the video frames

205 — Determining first markers in the PPG signal

207 — Determining second markers in the BCG signal

209 — Using a selected first marker of the first markers and/or a selected second marker of the second markers to define a trigger time for acquiring medical data from the subject

# Fig. 2

Fig. 3

$PPG_1$  $BCG_2$  $BCG_1$  $I_1$

$PPG_1$  $BCG_2$  $BCG_1$  $I_M$

# Fig. 4

Fig. 5

601 — Receiving video frames of a video which have been acquired from a subject

603 — Identifying in the frames first regions which are candidate sources of PPG information

605 — Determining for each first region a candidate PPG signal

607 — Selecting a subset of the set of candidate PPG signals based on their similarity with the BCG signal

609 — Combining the subset of candidate PPG signals to obtain the PPG signal

## Fig. 6

701 — Segmenting the PPG signal and the BCG signal into cardiac cycles of the subject

703 — Generating representative cycle waveforms from the segmented PPG signal and the BCG signal respectively

705 — Using the generated representative cycle waveforms to determine a pair of first marker and the second marker

707 — Using the pair for determining the temporal offset

## Fig. 7

Fig. 8

Fig. 9

Fig. 10

Fig. 11

Europäisches
Patentamt

European
Patent Office

Office européen
des brevets

# EUROPEAN SEARCH REPORT

**Application Number**

EP 23 18 5979

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WANG WENJIN ET AL: "Fundamentals of Camera-PPG Based Magnetic Resonance Imaging", IEEE JOURNAL OF BIOMEDICAL AND HEALTH INFORMATICS, IEEE, PISCATAWAY, NJ, USA, vol. 26, no. 9, 17 December 2021 (2021-12-17), pages 4378-4389, XP011919507, ISSN: 2168-2194, DOI: 10.1109/JBHI.2021.3136603 [retrieved on 2021-12-20] | 1,11,14, 15 | INV. A61B5/0205 A61B5/024 A61B5/11 A61B5/00 G01R33/567 ADD. A61B5/055 |
| A | * abstract; part I; figure 11 * | 2-4 | |
| A | US 2016/331239 A1 (MACLAREN JULIAN [US] ET AL) 17 November 2016 (2016-11-17) * paragraphs [0014], [0018], [0019]; claims 1, 8, 10, 12; figures 1, 4B, 4C * | 1 | |
| A | US 2020/046300 A1 (SENEGAS JULIEN [DE] ET AL) 13 February 2020 (2020-02-13) * paragraphs [0001], [0081], [0093]; claims 1, 2; figure 1 * | 1 | TECHNICAL FIELDS SEARCHED (IPC) A61B G01R |

-/--

The present search report has been drawn up for all claims

| Place of search | Date of completion of the search | Examiner |
|---|---|---|
| The Hague | 16 April 2024 | Fauché, Yann |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 23 18 5979

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SPICHER NICOLAI ET AL: "Initial evaluation of prospective cardiac triggering using photoplethysmography signals recorded with a video camera compared to pulse oximetry and electrocardiography at 7T MRI", BIOMEDICAL ENGINEERING ONLINE, vol. 15, no. 1, 1 December 2016 (2016-12-01), XP055870617, DOI: 10.1186/s12938-016-0245-3 Retrieved from the Internet: URL:https://www.researchgate.net/publication/310780429_Initial_evaluation_of_prospective_cardiac_triggering_using_photoplethysmography_signals_recorded_with_a_video_camera_compared_to_pulse_oximetry_and_electrocardiography_at_7T_MRI/fulltext/596e5619a6fdcc2416901294/Initial-evaluation-of-prospective-car> * abstract; figure 3 * | 1 | |
| A | US 2017/055934 A1 (HENNING ANDRE [DE]) 2 March 2017 (2017-03-02) * paragraph [0002]; claim 1; figures 2, 5 * | 1 | TECHNICAL FIELDS SEARCHED (IPC) |

The present search report has been drawn up for all claims

| Place of search | Date of completion of the search | Examiner |
|---|---|---|
| The Hague | 16 April 2024 | Fauché, Yann |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

    see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

    2-4, 11, 14, 15(completely); 1(partially)

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION**
**SHEET B**

The Search Division considers that the present European patent application does not comply with the requirements of unity of invention and relates to several inventions or groups of inventions, namely:

```
1. claims: 2-4, 11, 14, 15(completely); 1(partially)

       How to define a more accurate trigger time for the
       acquisition of medical data using remote camera-based
       measurements.
                                  - - -


2. claims: 5-10, 12, 13(completely); 1(partially)

       How to extract and separate a BCG signal and a PPG signal
       using remote camera-based measurements.
                                  - - -
```

## ANNEX TO THE EUROPEAN SEARCH REPORT
## ON EUROPEAN PATENT APPLICATION NO.

EP 23 18 5979

This annex lists the patent family members relating to the patent documents cited in the above-mentioned European search report.
The members are as contained in the European Patent Office EDP file on
The European Patent Office is in no way liable for these particulars which are merely given for the purpose of information.

16-04-2024

| Patent document cited in search report | Publication date | Patent family member(s) | Publication date |
|---|---|---|---|
| US 2016331239 A1 | 17-11-2016 | CN 106456045 A | 22-02-2017 |
| | | DE 112015000614 T5 | 27-10-2016 |
| | | JP 6426201 B2 | 21-11-2018 |
| | | JP 2017505697 A | 23-02-2017 |
| | | US 2016331239 A1 | 17-11-2016 |
| | | WO 2015117084 A1 | 06-08-2015 |
| US 2020046300 A1 | 13-02-2020 | CN 110446453 A | 12-11-2019 |
| | | EP 3375354 A1 | 19-09-2018 |
| | | EP 3595513 A1 | 22-01-2020 |
| | | JP 7319192 B2 | 01-08-2023 |
| | | JP 2020511222 A | 16-04-2020 |
| | | JP 2023100634 A | 19-07-2023 |
| | | US 2020046300 A1 | 13-02-2020 |
| | | WO 2018167275 A1 | 20-09-2018 |
| US 2017055934 A1 | 02-03-2017 | CN 106485046 A | 08-03-2017 |
| | | DE 102015216115 A1 | 02-03-2017 |
| | | US 2017055934 A1 | 02-03-2017 |

EPO FORM P0459

For more details about this annex : see Official Journal of the European Patent Office, No. 12/82